# EUROPÄISCHE PATENTSCHRIFT

(11) **EP 1 742 623 B1**
(45) Veröffentlichungstag und Bekanntmachung des Hinweises auf die Patenterteilung: **21.07.2010**
(21) Anmeldenummer: 05747555.0
(22) Anmeldetag: 02.05.2005
(51) Int. Cl.: A61K 31/00, A61K 31/425, A61P 35/00, A61K 31/282, A61K 31/53, A61K 31/475

(54) **KOMBINATIONSPRÄPARATE ENTHALTEND 2-METHYLTHIAZOLIDIN-2,4-DICARBONSÄURE**
2-METHYLTHIAZOLIDINE-2,4-DICARBOXYLIC ACID-CONTAINING COMBINATION PREPARATIONS
PREPARATION COMBINEE CONTENANT DE L'ACIDE 2-METHYLTHIAZOLIDIN-2,4-DICARBOXYLIQUE

(30) Priorität: 03.05.2004 DE 102004021658; 10.05.2004 US 569570 P
(43) Veröffentlichungstag der Anmeldung: 17.01.2007
(73) Patentinhaber: Susilo, Rudy, Dr., 50999 Köln (DE)
(72) Erfinder: SUSILO, Rudy, 50999 Köln (DE); AMTMANN, Eberhard, 69121 Heidelberg (DE)
(74) Vertreter: Arth, Hans-Lothar
(86) Internationale Anmeldenummer: PCT/DE2005/000810
(87) Internationale Veröffentlichungsnummer: WO 2005/105062

(56) Entgegenhaltungen:
- WO-A-01/60343
- FR-A- 2 133 508
- WLODEK ET AL: "2-Methyl-thiazolidine-2,4-dicarboxylic acid protects against paracetamol induced toxicity in human liver derived HepG2 cells" ACTA BIOCHIMICA POLONICA, POLISH SCIENTIFIC PUBLISHERS, WARSAW, PO, Bd. 4, Nr. 44, Oktober 1997 (1997-10), Seiten 759-66, XP002075765 ISSN: 0001-527X
- SOKOLOWSKA, MARIA ET AL: "Protective effect of .alpha.-keto acids on the oxidative hemolysis" POLISH JOURNAL OF PHARMACOLOGY , 51(5), 429-434 CODEN: PJPAE3; ISSN: 1230-6002, 1999, XP008057282
- WLODEK, L. ET AL: "2-Methyl-thiazolidine-2,4-dicarboxylic acid as prodrug of L-cysteine. Protection against paracetamol hepatotoxicity in mice" FUNDAMENTAL & CLINICAL PHARMACOLOGY , 11(5), 454-459 CODEN: FCPHEZ; ISSN: 0767-3981, 1997, XP008057290
- WLODEK L: "Biological properties of 2-methyl-thiazolidine-2,4-dicarboxylic acid, the product of nonenzymatic condensation of L-cysteine with pyruvate" AMINO ACIDS (VIENNA), Bd. 17, Nr. 1, 1999, Seite 71, XP008057284 & 6TH INTERNATIONAL CONGRESS ON AMINO ACIDS; BONN, GERMANY; AUGUST 3-7, 1999 ISSN: 0939-4451
- WLODEK, LIDIA ET AL: "The modulation of IL-2 dependent proliferation of CTLL-2 cells by 2-methyl-thiazolidine-2,4-dicarboxylic acid" IMMUNOPHARMACOLOGY , 30(1), 51-8 CODEN: IMMUDP; ISSN: 0162-3109, 1995, XP008057299
- HIDAKA ET AL., CANCER RESEARCH, 38 S. 4650 - 4653, 1978: 1978,
- NAKASHIMA ET AL., CANCER RESEARCH, 34,3258-3261, 1974:
- ALAS ET AL., ANTICANCER RES., 20, 2961 - 2966, 2000:
- OSHISHI ET AL., CANCER CHEMOTHER. PHARMACOL., 38, 446 -452, 1996:
- BUDD ET AL., CANCER, 80 (6), 1134 - 1140,1997:

## Beschreibung

Die vorliegende Erfindung betrifft die Verwendung von 2-Methylthiazolidin-2,4-dicarbonsäure und/oder physiologisch verträgliche Salze davon zur Herstellung eines Arzneistoffes zur Steigerung der Wirksamkeit von Cisplotin, Temozolomid und Vincristin bei der Krebsbehandlung.

Die Synthese von 2-Methylthiazolidin-2,4-dicarbonsäure ist aus DE-OS 21 16 629 bekannt. EP 0 969 834 B1 offenbart die Verwendung von 2-Methylthiazolidin-2,4-dicarbonsäure als Mukolytikum, EP 98 916 809 beschreibt ein Kombinationspräparat aus 2-Methylthiazolidin-2,4-dicarbonsäure und Paracetamol und EP 01 915 023 behandelt die Verwendung von 2-Methylthiazolidin-2,4-dicarbonsäure zur Behandlung von Infektionskrankheiten, insbesondere HIV.

Das europäische Patent EP 1 255 538 B1 offenbart die Verwendung von 2-Methylthiazolidin-2,4-dicarbonsäure zur Behandlung und Prophylaxe von Krebserkrankungen. In diesem Patent wird auch die Verwendung von 2-Methylthiazolidin-2,4-dicarbonsäure zur Herstellung einer Medikaments zur Vermeidung und/oder Reduzierung von unerwünschten Nebenwirkungen von Zytostatika beschrieben.

Zytostatika sind Substanzen, die den Eintritt der Kern- und/oder Plasmateilung (Karyo- bzw. Zytokinese) verhindern oder erheblich verzögern bzw. ihren Ablauf unterbrechen, stören. Sie greifen entweder in die Reduplikation oder Transkription der DNS oder in die Ausbildung und Trennung deren Trägerstrukturen ein und führen zu teilungsstörenden Chromosomenaberrationen oder unterdrücken die Ausbildung bzw. stören die Funktion des Spindelapparates (und sind fast stets mutagen). Im weiteren Sinne sind Zytostatika auch Substanzen, die die Bereitstellung der für die Nucleoproteinsynthese oder die Spindelfunktion notwendige Energie stören bzw. verhindern. Die Einteilung erfolgt in der Regel aufgrund des Wirkmechanismus in Antimetaboliten, Alkylanzien, zytostatisch wirkende Antibiotika und Mitosehemmer (Roche Lexikon Medizin, 4.Auflage; © Urban & Fischer Verlag, München 1984/1987/1993/1999).

EP 1 255 538 B1 offenbart die Verwendung von 2-Methylthiazolidin-2,4-dicarbonsäure zur Reduktion von unerwünschten Nebenwirkungen von Zytostatika. EP 1 255 538 B1 löst somit das Problem, wie unerwünschte Nebenwirkungen von Zytostatika reduziert werden können.

Aufgabe der vorliegenden Erfindung ist es hingegen, die Wirkung, d.h. die Wirksamkeit und/oder Aktivität von Cisplatin, Temozolomid und Vincristin zu steigern.

Diese Aufgabe wird durch die technische Lehre der unabhängigen Patentansprüche gelöst. Vorteilhafte Ausgestaltungen der Erfindung sind in den abhängigen Patentansprüchen, der Beschreibung sowie den Beispielen angegeben.

Überraschenderweise hat sich gezeigt, dass die oben beschriebene Wirkung von Cisplatin, Temozolomid und Vincristin durch die Verabreichung von 2-Methylthiazolidin-2,4-dicarbonsäure gesteigert werden kann.

Somit betrifft die vorliegende Erfindung Kombinationspräparate, welche 2-Methylthiazolidin-2,4-dicarbonsäure und/oder physiologisch verträgliche Salze von dieser Verbindung und Cisplatin, Temozolomid oder Vincristin enthalten.

2-Methylthiazolidin-2,4-dicarbonsäure ist eine chemische Verbindung mit zwei stereogenen Zentren, wobei die Position 4 insbesondere bevorzugt die R-Konfiguration aufweist. Das stereogene Zentrum an Position 2 besitzt keine bevorzugte Konfiguration.

Die Bezeichnung 2-Methylthiazolidin-2,4-dicarbonsäure soll für die diastereomeren Verbindungen (2*R*,4*R*)-2-Methylthiazolidin-2,4-dicarbonsäure, (2*S*,4*R*)-2-Methylthiazolidin-2,4-dicarbonsäure sowie (2*RS*,4*R*)-2-Methylthiazolidin-2,4-dicarbonsäure stehen. Bei (2RS,4R)-2-Methyl-thiazolidin-2,4-dicarbonsäure kann das Molverhältnis von (2*R*,4*R*)-2-Methylthiazolidin-2,4-dicarbonsäure zu (2*S*,4*R*)-2-Methylthiazolidin-2,4-dicarbonsäure 90 : 10 bis 10 : 90 betragen, wobei bevorzugt der Anteil an (2*R*,4*R*)-2-Methylthiazolidin-2,4-dicarbonsäure überwiegt.

Ferner soll die Bezeichnung 2-Methylthiazolidin-2,4-dicarbonsäure nicht nur die freie Säure, sondern auch physiologisch verträgliche Salze umfassen. Demnach kann die Verbindung 2-Methylthiazolidin-2,4-dicarbonsäure bzw. ein Enantiomer, Diastereomer oder Enantiomeren- und/oder Diastereomerengemisch davon als freie Säure und/oder in Form des pharmakologisch verträglichen Salzes verabreicht werden. Geeignete Beispiele dieser Salze umfassen Säureadditionssalze und Alkalimetallsalze. So können Alkalimetallsalze wie das Natriumsalz, das Kaliumsalz, das Lithiumsalz, das Magnesiumsalz, das Calciumsalz und/oder Alkylammoniumsalze eingesetzt werden. Als Säuren, welche ein Säureadditionssalz bilden, können die folgenden genannt werden: Schwefelsäure, Sulfonsäure, Phosphorsäure, Salpetersäure, salpetrige Säure, Perchlorsäure, Bromwasserstoffsäure, Chlorwasserstoffsäure, Ameisensäure, Essigsäure, Propionsäure, Bernsteinsäure, Oxalsäure, Gluconsäure (Glycons., Dextronsäure), Milchsäure, Apfelsäure, Weinsäure, Tartronsäure (Hydroxymalonsäure, Hydroxypropandisäure), Fumarsäure, Zitronensäure, Ascorbinsäure, Maleinsäure, Malonsäure, Hydroxymaleinsäure, Brenztraubensäure, Phenylessigsäure, (o-, m-, p-) Toluylsäure, Benzoesäure, p-Aminobenzoesäure, p-Hydroxybenzoesäure, Salicylsäure, p-Aminosalicylsäure, Methansulfonsäure, Ethansulfonsäure, Hydroxyethansulfonsäure, Ethylensulfonsäure, p-Toluolsulfonsäure, Naphthylsulfonsäure, Naphthylaminsulfonsäure, Sulfanilsäure, Camphersulfonsäure, Chinasäure (Chininsäure), o-Methyl-mandelsäure, Hydrogenbenzolsulfonsäure, Pikrinsäure (2,4,6-Trinitrophenol), Adipinsäure, d-o-Tolylweinsäure. Auch Aminosäuren können für die Salzbildung eingesetzt werden, wie beispielsweise Glycin, Alanin, Valin, Leucin, Isoleucin, Serin, Threonin, Phenylalanin, Tyrosin, Tryptophan, Lysin, Arginin, Histidin, Aspartat, Glutamat, Asparagin, Glutamin, Cystein, Methionin und Prolin, wobei die Aminosäuren Methionin, Tryptophan, Lysin und Arginin bevorzugt sind.

Ein Kombinationspräparat, welches 2-Methylthiazolidin-2,4-dicarbonsäure, d.h. mindestens ein Enantiomer oder ein Diastereomer von 2-Methylthiazolidin-2,4-dicarbonsäure als freie Säure oder als Salz, und Cisplatin, Temozolomid oder Vincristin enthält, muss beide Wirkstoffe nicht zwingend in einer pharmazeutischen Formulierung enthalten.

Natürlich können pharmazeutische Formulierungen bereitgestellt werden, welche zugleich 2-Methylthiazolidin-2,4-dicarbonsäure und Cisplatin, Temozolomid oder Vincristin enthalten. Bevorzugt sind Kombinationspräparate, welche zwei getrennte pharmazeutische Formulierungen umfassen, nämlich eine für 2-Methylthiazolidin-2,4-dicarbonsäure und eine weitere für Cisplatin, Temozolomid oder Vincristin. Dies hat den Vorteil, dass zwar beide Wirkstoffe zeitgleich appliziert werden können, jedoch die verabreichten Mengen beider Verbindungen sowie die Zeitpunkte der Applikation beider Verbindungen variiert werden können. Ein weiterer Vorteil besteht darin, die nebenwirkungsarme Verbindung 2-Methylthiazolidin-2,4-dicarbonsäure vor, gleichzeitig oder nach der Gabe von Cisplatin, Temozolomid oder Vincristin zu verabreichen. Vor der Gabe von Cisplatin, Temozolomid oder Vincristin kann 6h, 12h, 18h, 24h, 30h, 36h, 42h, 48h, 54h oder 60h vorher bedeuten. Unter dem Begriff gleichzeitig soll sowohl die Gabe beider Wirkstoffe in einer einzigen Formulierung als auch eine bis zu einer Stunde zeitversetzte Applikation eines Wirkstoffs verstanden werden. Nach der Gabe von Cisplatin, Temozolomid oder Vincristin kann 6h, 12h, 18h, 24h, 30h, 36h, 42h, 48h, 54h oder 60h nachher bedeuten. Bevorzugt ist eine Verabreichung von 2-Methylthiazolidin-2,4-dicarbonsäure nach der Gabe von Cisplatin, Temozolomid oder Vincristin und insbesondere bevorzugt 24h oder 48h danach.

Somit umfasst das Kombinationspräparat vorzugsweise eine pharmazeutische Formulierung enthaltend 2-Methylthiazolidin-2,4-dicarbonsäure und/oder physiologisch verträgliche Salze davon sowie mindestens eine weitere pharmazeutische Formulierung enthaltend Cisplatin, Temozolomid oder Vincristin. Erfindungsgemäß ist eine Kombination von 2-Methylthiazolidin-2,4-dicarbonsäure und/oder Salzen von 2-Methylthiazolidin-2,4-dicarbonsäure mit Cisplatin, Temozolomid und/oder Vincristin.

Die pharmazeutische Formulierung enthaltend 2-Methylthiazolidin-2,4-dicarbonsäure und die pharmazeutische Formulierung enthaltend Cisplatin, Temozolomid oder Vincristin können des weiteren mindestens einen physiologisch verträglichen Träger, Zusatzstoff, Hilfsstoff und/oder Lösungsmittel enthalten.

Die pharmazeutischen Formulierungen werden mit den üblichen festen oder flüssigen Trägerstoffen oder Verdünnungsmitteln und den üblicherweise verwendeten pharmazeutischen Hilfsstoffen entsprechend der gewünschten Applikationsart mit einer geeigneten Dosierung in bekannter Weise hergestellt. Solche Darreichungsformen sind beispielsweise Tabletten, Filmtabletten, Schichttabletten, Dragees, Kapseln, Mikrokapseln, Mikropellets und Pellets, Pillen, Granulaten, Pulver, Puder, Lösungen, Dispersionen, Suspensionen Suppositorien, Emulsionen, Dispersionen, Zäpfchen, Gelen, Salben, Sirup oder Depotformen oder Inhalationslösungen bzw. Inhalationspulver. Zudem umfassen die pharmazeutischen Zusammensetzungen Formulierungen wie Schichttabletten zur kontrollierten und/oder kontinuierlichen Freisetzung des Wirkstoffs sowie Mikroverkapselungen als spezielle Darreichungsform.

Derartige Zubereitungen sind unter anderem für die Inhalation oder die intravenöse, intraperitoneale, intramuskuläre, subkutane, orale, rektale, transdermale, topikale, intradermale, intragastrale, intrakutane, intravaginale, intranasale, intrabuccale, perkutane oder sublinguale Verabreichung geeignet.

Besonders vorteilhafte Darreichungsformen sind die orale und topische Applikation, die Injektion als auch die Inhalation.

Entsprechende Tabletten können beispielsweise durch Mischen der erfindungsgemäß einsetzbaren Verbindung und/oder deren Salz mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem gemäß Anspruch 1 einsetzbaren Wirkstoff können zusätzlich geschmacksverbessernde Mittel wie Saccharin, Cyclamat oder Zucker sowie Aromastoffe wie Vanillin oder Orangenextrakt enthalten. Sie können außerdem Suspendierhilfsstoffe wie Natriumcarboxymethylcellulose oder Konservierungsmittel wie p-Hydroxybenzoate enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyethylenglykol bzw. Derivaten davon herstellen.

Derartige Formulierungen sind unter anderem für die Inhalation oder die intravenöse, intraperitoneale, intramuskuläre, subkutane, orale, rektale, transdermale, topikale, intradermale, intragastrale, intrakutane, intravaginale, intranasale, intrabuccale, perkutane oder sublinguale Verabreichung geeignet.

Als pharmakologisch verträgliche Träger können beispielsweise Lactose, Stärke, Sorbitol, Sucrose, Cellulose, Magnesiumstearat, Dicalciumphosphate, Calciumsulfate, Talk, Mannitol, Ethylalcohol und dergleichen eingesetzt werden. Puder als auch Tabletten können zu 5 bis 95% aus einem derartigen Träger bestehen.

Als Bindemittel können zudem Stärke, Gelatine, natürliche Zucker, natürliche als auch synthetische Gummis wie beispielsweise Akaziengummi oder Guar-Gummi, Natriumalginat, Carboxymethyl-Cellulose, Polyethylenglycol und Wachse eingesetzt werden. Als Gleitmittel können Borsäure, Natriumbenzoat, Natriumacetat, Natriumchlorid, und dergleichen dienen.

Ferner können den pharmazeutischen Zusammensetzungen noch Sprengmittel, Farbstoffe, Geschmacksstoffe und/oder Bindemittel zugesetzt werden.

Flüssige Formulierungen umfassen Lösungen, Suspensionen, Sprays und Emulsionen. Beispielsweise Injektionslösungen auf Wasserbasis oder Wasser-Propylenglycol-Basis für parenterale Injektionen.

Für die Zubereitung von Suppositorien werden bevorzugt niedrigschmelzende Wachse, Fettsäureester und Glyceride eingesetzt.

Kapseln werden beispielsweise aus Methylcellulose, Polyvinylalcohole oder denaturierte Gelatine oder Stärke hergestellt.

Als Sprengmittel können Stärke, Natrium-Carboxymethylstärke, natürliche und synthetische Gummis wie beispielsweise Johannisbrotkernmehl, Karaya, Guar, Tragacanth und Agar sowie Cellulosederivate wie Methylcellulose, Natrium-Carboxymethylcellulose, microkristalline Cellulose sowie Alginate, Tonerden und Bentonite verwendet werden. Diese Bestandteile können in Mengen von 2 bis 30 Gew.-% eingesetzt werden.

Als Bindemittel können Zucker, Stärke aus Korn, Reis oder Kartoffeln, natürliche Gummis wie Akaziengummi, Gelatine, Tragacanth, Alginsäure, Natriumalginat, Ammonium-Calcium-Alginat, Methylcellulose, Natrium-Carboxymethylcellulose, Hydroxypropyl-methylcellulose, Polyvinylpyrrolidon sowie anorganische Verbindungen wie Magnesium-Aluminum-Silicate zugesetzt werden. Die Bindemittel könne in Mengen von 1 bis 30 Gew.-% zugesetzt werden.

Als Gleitmittel können Stearate wie Magnesiumstearat, Calciumstearat, Kaliumstearat, Stearinsäure, hochschmelzende Wachse als auch wasserlösliche Gleitmittel wie Natriumchlorid, Natriumbenzoat, Natriumacetat, Natrioumoleat, Polyethylenglycol und Aminosäuren wie Leucin eingesetzt werden. Derartige Gleitmittel können in Mengen von 0,05 bis 15 Gew.-% verwendet werden.

Der Arzneistoff wird insbesondere für die Steigerung der Wirksamkeit von Zytostatika in der Krebstherapie eingesetzt. Die Indikation richtet sich vor allem nach der Art des verwendeten wirkstoffs gemäß Anspruch 1, d.h. den Krebsarten, wo dieser wirkstoff bisher als Einzelsubstanz eingesetzt worden ist.

Erfindungsgemäß kann der Arzneistoff für die Steigerung der Wirksamkeit von Cisplatin, Temozolomid oder Vincristin bei folgenden Tumoren bzw. Krebsarten eingesetzt werden: akute und chronische myeloische Leukämie, akute und chronische lymphatische Leukämie, Analkarzinom, Astrozytom, Basaliom, kleinzelliges und nichtkleinzelliges Bronchialkarzinom, Burkitt-Lymphom, CUP-Syndrom, Dünndarmtumore, Endometriumkarzinom, Ependymom, Ewing-Sarkom, Gallenblasen- und Gallengangkarzinom, Glioblastom, Haarzell-Leukämie, Hirntumoren (Gliome), Hirnmetastasen, Hodenkrebs, Morbus Hodgkin, Hypophysentumore, Karzinoide, Kaposi-Sarkom, Kehlkopfkrebs, Keimzellentumor, Knochenkrebs, Kopf-Hals-Tumore, Kolonkarzinom, Kraniopharyngeome, Krebs im Mundbereich und auf der Lippe, Leberzellkarzinom, Lebermetastasen, Lidtumor, Magenkrebs, Malignes Melanom, Mammakarzinom, Medulloblastome, Meningeome, Neurinom, Nierenzellkarzinom, Non-Hodgkin-Lymphome, Oligodendrogliom, Ösophaguskarzinom, Osteosarkom, Ovarial-Karzinom, Pankreaskarzinom, Peniskarzinom, Plasmozytom, Prostatakarzinom, Rektumkarzinom, Retinoblastom, Schilddrüsenkarzinom, Spinaliom, Thymom, Tubenkarzinom, Tumoren des Auges, Urethrakrebs, Urothelkarzinom, Vulvakarzinom, Weichteiltumoren, Wilms Tumor, Zervixkarzinom und Zungenkrebs.

### Beispiele

### Beispiel 1:

Untersucht wurde die in vitro Zytotoxizität von 2-Methylthiazolidin-2,4-dicarbonsäure und N-Acetylcystein in humanen Tumorzelllinien.

### Verfahren:

Eingesetzt wurden 2-Methylthiazolidin-2,4-dicarbonsäure (MTDC), hergestellt bei Trommsdorff GmbH & Co. KG, Alsdorf (MTDC: 40 mg/ml in Wasser dd),
N-Acetylcystein 40 mg/ml in Wasser,
Temozolomid 5 mg/ml in DMSO.

### Zellkultur:

Die Zellen wurden in RPMI 1640 Medium (Sigma, München) mit 10%igem FCS (Sigma, München) in einer 50 ml Kulturflasche (Greiner) bei 37°C in einer 5%igen CO₂ Atmosphäre aufgenommen und wurden ein bis zwei Mal wöchentlich dekantiert.

### Verwendete Zelllinien:

| | |
|---|---|
| Neurogliom-Zellen | H4 |
| Blasenkarzinom-Zellen | EJ |

### Zytotoxizitäts-Assay:

Monoschichten von Tumorzellen wurden durch Dispergierung von 2 x 10⁶ Tumorzellen in 96 Lochplatten (Greiner) zubereitet. Nach einer Inkubationszeit von 24h wurde das Medium durch ein Medium ausgetauscht, das die Testverbindungen enthielt. Sämtliche Assays wurden 4-fach durchgeführt.

Nach 72h wurde die Anzahl lebender Zellen durch Anfärbung mittels Kristallviolett bestimmt.

Das Medium wurde dazu aus jedem Loch der Platte entfernt, die Platten wurden zweimal mit PBS gewaschen, die Zellen mittels PBS enthaltend 1 % Paraformaldehyd fixiert und 15 Minuten bei Raumtemperatur getrocknet. Die Platten wurden dann zweimal mit PBS gespült und bei Raumtemperatur getrocknet. Die Zellen wurden anschließend für zwei Minuten mit 1%iger Kristallviolettlösung in Wasser gefärbt und nach dem Waschen mit Wasser wurde die optische Dichte in einem ELISA-Spektrometer bei 595 nm bestimmt. Die Durchschnittswerte und Standardabweichungen für die Durchschnittswerte wurden für alle vier Parallelversuche bestimmt.

### Ergebnisse:

In den humanen Gliomzellen H4 zeigte 2-Methylthiazolidin-2,4-dicarbonsäure einen signifikanten zytotoxischen Effekt. Ein IC₅₀-Wert von 9,66 µg<ml wurde gefunden. Hingegen zeigte N-Acetylcystein bis hin zu relativ hohen eingesetzten Konzentrationen keine Wirkung.

Das Wachstum der Blasenkarzinom-Zellline EJ wurde ferner nicht durch 2-Methylthiazolidin-2,4-dicarbonsäure beeinflußt.

Wenn jedoch 2-Methylthiazolidin-2,4-dicarbonsäure zusammen mit dem Zytostatikum Temozolomid verabreicht wurde, zeigt sich eine deutlich gesteigerte Empfindlichkeit der EJ-Zellen gegenüber Temozolomid in Vergleich zu den Versuchen mit Temozolomid bei Anwesenheit von 2-Methylthiazolidin-2,4-dicarbonsäure. Der IC₅₀-Wert für Temozolomid alleine betrug 1053,36 µg/ml. In der Gegenwart von 200 µg/ml 2-Methylthiazolidin-2,4-dicarbonsäure sank der IC₅₀-Wert von Temozolomid auf 96,58 µg/ml.

Im Gegensatz dazu brachte die Anwesenheit von 1000 µg/ml N-Acetylcystein keine Steigerung der Wirkung von Temozolomid auf die EJ-Zellen (IC₅₀-Wert von Temozolomid alleine betrug 1053,36 µg/ml; IC₅₀-Wert von Temozolomid in Gegenwart von 1000 µg/ml N-Acetylcystein betrug 903,24 µg/ml).

Diese Versuchsdaten belegen die unerwartete Steigerung der Wirksamkeit von Kombinationen aus einem Zytostatikum mit 2-Methylthiazolidin-2,4-dicarbonsäure gegenüber der alleinigen Verabreichung eines Zytostatikums und die Nichtbeeinflussung oder kaum eine Beeinflussung der Wirksamkeit eines Zytostatikums oder eines zytotoxischen oder anti-angiogenen Wirkstoffs durch N-Acetylcystein.

### Beispiel 2

### Verwendete Substanzen:

Natriumsalz der 2-Methylthiazolidin-2,4-dicarbonsäure (MTDC-Na) (Trommsdorff GmbH & Co. KG Arzneimittel);
   - Cisplatin (kommerziell erhältlich von Alfa);
   - Temozolomid (kommerziell erhältlich von Essex Pharma);
   - Vincristin (kommerziell erhältlich von Medac).

Folgende Stammlösungen wurden verwendet:
- MTDC-Na 40 mg/ml (gelöst in destilliertem Wasser),
- Cisplatin, 0,5 mg/ml (gelöst in destilliertem Wasser),
- Temozolomid, 5 mg/ml (gelöst in DMSO),
- Vincristin 1 mg/ml (gelöst in destilliertem Wasser).

Folgende Zelllinien wurden untersucht:
- Neurogliom H4
- Blasenkarzinom EJ
- Prostatakarzinom DU145

### Zellkulturen:

Die Zellen wurden in Kulturmedium RPMI 1640 (Sigma, München) und zusätzlich 10 % FCS (Sigma, München) in 50 ml Zellkultur-Fläschen (Greiner) bei 37°C sowie in einer 5% CO₂ Atmosphäre gehalten.

### Zytotoxizitäts-Assay:

Monolayer der Tumorzellen wurden durch Dispensierung von 2 x 106 Tumorzellen in 96-Lochplatten (Greiner) hergestellt.

Nach einer Inkubationszeit von 30 Stunden (t=0) wurde das Medium durch ein Medium ersetzt, welches das Anti-Krebs-Präparat enthielt.

Nach einer Inkubationszeit von weiteren 24 Stunden (t= +24h) wurde das Anti-Krebs-Mittel entfernt und das Medium ersetzt.

MTDC-Na wurde den Zellen zugefügt durch Austausch des vorhandenen Mediums durch ein frisches, MTDC-Na enthaltendes Medium.

Alle Untersuchungen wurden in dreifacher Ausfertigung durchgeführt.

Folgende Konzentrationen des Anti-Krebs-Präparates wurden verwendet:
- Temozolomid in einer Konzentration von 0-1000 µg/ml,
- Vincristin in einer Konzentration von 0-200 ng/ml,
- Cisplatin in einer Konzentration von 0-25 µg/ml.

Nach 126 Stunden Inkubation wurden die Zellen gesammelt und der Anteil der lebenden Zellen wurde durch Färbung mittels Kristallviolett bestimmt.

Dazu wurde das Medium entfernt, die Lochplatten zweimal mit PBS gewaschen, und anschließend wurden die Zellen mit einer PBS-Lösung enthaltend 1 % Paraformaldehyd für 15 Minuten bei Raumtemperatur fixiert. Die Platten wurden dann zweimal mit PBS gespült und bei Raumtemperatur getrocknet. Die Zellen wurden für zwei Minuten mit einer Lösung von 1 % Kristallviolett in Wasser angefärbt.

Nach dem Waschen mit Wasser wurde die optische Dichte durch einen ELISA-Reader bei 595 nm bestimmt. Die Mittelwerte und die Standardabweichungen der Mittelwerte wurden für die drei Versuchsreihen bestimmt.

IC₅₀-Werte wurden durch die Auftragung der Konzentration des Wirkstoffs versus die optische Dichte bestimmt.

Die Ergebnisse sind in Tabelle 1 wiedergegeben:

**Tabelle 1: Erhöhung der zytotoxischen Aktivität von MTDC-Na bei einer Kombination mit einer anderen zytotoxischen und/oder zytostatischen Verbindung**

| **Zelllinie** | **zytotoxische und/oder zytostatische Verbindung** | **Erhöhung der Aktivität Reduktion des IC₅₀-Wertes** |
|---|---|---|
| Prostatakarzinom **DU 145** | Cisplatin / MTDC-Na (1000 µg/ml) | 2 fach |
| Prostatakarzinom **DU 145** | Vincristin / MTDC-Na (1000 µg/ml) | 2.7 fach |
| Blasenkarzinom **EJ** | Cisplatin / MTDC-Na (1000 µg/ml) | 10 fach |
| Blasenkarzinom **EJ** | Temozolomid / MTDC-Na (1000 µg/ml) | 67 fach |
| Blasenkarzinom **EJ** | Vincristin / MTDC-Na (1000 µg/ml) | 6 fach |
| Blasenkarzinom **EJ** | Vincristin / MTDC-Na (1000 µg/ml) | 4.8 fach |
| Neurogliom **H4** | Cisplatin / MTDC-Na (200 µg/ml) | 4 fach |
| Neurogliom **H4** | Temozolomid / MTDC-Na (200 µg/ml) | 15 fach |

## Patentansprüche

1. Verwendung von 2-Methylthiazolidin-2,4-dicarbonsäure und/oder physiologisch verträgliche Salze davon zur Herstellung eines Arzneistoffes zur Steigerung der Wirksamkeit von Cisplatin, Temozolomid und Vincristin bei der Krebsbehandlung.

2. Verwendung gemäß Anspruch 1, wobei es sich bei den Krebserkrankungen um akute und chronische myeloische Leukämie, akute und chronische lymphatische Leukämie, Analkarzinom, Astrozytom, Basaliom, kleinzelliges und nichtkleinzelliges Bronchialkarzinom, Burkitt-Lymphom, CUP-Syndrom, Dünndarmtumore, Endometriumkarzinom, Ependymom, Ewing-Sarkom, Gallenblasen- und Gallengangkarzinom, Glioblastom, Haarzell-Leukämie, Hirntumoren (Gliome), Hirnmetastasen, Hodenkrebs, Morbus Hodgkin, Hypophysentumore, Karzinoide, Kaposi-Sarkom, Kehlkopfkrebs, Keimzellentumor, Knochenkrebs, Kopf-Hals-Tumore, Kolonkarzinom, Kraniopharyngeome, Krebs im Mundbereich und auf der Lippe, Leberzellkarzinom, Lebermetastasen, Lidtumor, Magenkrebs, Malignes Melanom, Mammakarzinom, Medulloblastome, Meningeome, Neurinom, Nierenzellkarzinom, Non-Hodgkin-Lymphome, Oligodendrogliom, Ösophaguskarzinom, Osteosarkom, Ovarial-Karzinom, Pankreaskarzinom, Peniskarzinom, Plasmozytom, Prostatakarzinom, Rektumkarzinom, Retinoblastom, Schilddrüsenkarzinom, Spinaliom, Thymom, Tubenkarzinom, Tumoren des Auges, Urethrakrebs, Urothelkarzinom, Vulvakarzinom, Weichteiltumoren, Wilms Tumor, Zervixkarzinom und Zungenkrebs handelt.

## Claims

1. Use of 2-methylthiazolidine-2,4-dicarboxylic acid and/or physiologically acceptable salts thereof for the manufacture of a therapeutic agent for increasing the efficacy of cisplatin, temozolomide and vincristine in cancer treatment.

2. Use according to claim 1, wherein the cancer diseases are acute and chronic myeloid leukemia, acute and chronic lymphatic leukemia, anal carcinoma, astrocytoma, basal cell carcinoma, small cell and non small cell bronchial carcinoma, Burkitt's lymphoma, CUP syndrome, small intestine tumors, endometrial carcinoma, ependymoma, Ewing's tumors, gall bladder and bile duct carcinoma, glioblastoma, hairy cell leukemia, brain tumors (gliomas), brain metastases, testicle cancer, Hodgkin's disease, hypophysis tumors, carcinoids, Kaposi's sarcoma, laryngeal cancer, germ cell tumor, bone cancer, head and neck tumors, colon carcinoma, craniopharyngiomas, cancer in the mouth area and on the lip, liver cell carcinoma, liver metastases, eyelid tumor, stomach cancer, malignant melanoma, breast carcinoma, medulloblastomas, meningiomas, neurinoma, renal cell carcinoma, Non-Hodgkin's lymphomas, oligodendroglioma, esophageal carcinoma, osteosarcoma, ovarian carcinoma, pancreatic carcinoma, penile carcinoma, plasmocytoma, prostate carcinoma, rectal carcinoma, retinoblastoma, thyroid carcinoma, spinalioma, thymoma, tube carcinoma, eye tumors, urethral cancer, urothelial carcinoma, vulva carcinoma, soft tissue tumors, Wilms' tumor, cervical carcinoma and tongue cancer.

## Revendications

1. Utilisation d'acide 2-méthylthiazolidine-2,4-dicarboxylique et/ou ses sels physiologiquement acceptables pour la fabrication d'une substance thérapeutique pour augmenter l'efficacité de cisplatine, témozolomide et vincristine pour le traitement de cancer.

2. Utilisation selon la revendication 1, où les maladies cancéreuses sont leucémie myéloïde aigüe et chronique, leucémie lymphoïde aigüe et chronique, carcinome anal, astrocytome, carcinome basocellulaire, carcinome bronchique à petite cellule et non à petite cellule, lymphome de Burkitt, CUP syndrome, tumeurs de l'intestin grêle, carcinome endométrial, épendymome, sarcome d'Ewing, carcinome de la vésicule biliaire et de la voie biliaire, glioblastome, tricholeucémie, tumeurs de cerveau (gliomes), métastases cérébrales, cancer du testicule, maladie de Hodgkin, tumeurs de l'hypophyse, tumeurs carcinoïdes, sarcome de Kaposi, cancer du larynx, tumeur germinale, cancer des os, tumeurs en ORL et des VADS, carcinome du côlon, craniopharyngiomes, cancer buccal et de la lèvre, carcinome hépatocellulaire, métastases hépatiques, tumeur de la paupière, cancer de l'estomac, mélanome malin, carcinome du sein, médulloblastomes, méningiomes, neurinome, carcinome à cellules rénales, lymphomes non-hodgkiniens, oligodendrogliome, carcinome de l'oesophage, ostéosarcome, carcinome ovarien, carcinome du pancréas, carcinome du pénis, plasmocytome, carcinome de la prostate, carcinome du rectum, rétinoblastome, carcinome de la thyroïde, spinaliome, thymome, carcinome tubaire, tumeurs de l'oeil, cancer de l'urètre, carcinome urothélial, carcinome de la vulve, tumeurs des tissus mous, tumeur de Wilms, carcinome cervical et cancer de la langue.
